# EUROPEAN PATENT APPLICATION

(11) **EP 1 798 219 A1**
(43) Date of publication of application: **20.06.2007**
(21) Application number: 06026033.8
(22) Date of filing: 15.12.2006
(51) Int. Cl.: C07C 237/46, A61K 31/167, A61K 49/04

(54) **Contrast agents**

(30) Priority: 15.12.2005 NO 20055980
(71) Applicant: GE Healthcare AS, Nydalen 0401 Oslo (NO)
(72) Inventor: Wistrand, Lars-Gören GE Healthcare AS, 0401 Oslo (NO); Thaning, Mikkel GE Healthcare AS, 0401 Oslo (NO); Axelsson, Oskar GE Healthcare AS, 0401 Oslo (NO); Andersson, Sven, 23433 Lomma (SE)
(74) Representative: Flechsler, Insa

(57) **Abstract**

The present invention relates to a class of compounds and to diagnostic compositions containing such compounds where the compounds are iodine containing compounds. More specifically the iodine containing compounds are chemical compounds containing a benzene scaffolding moiety allowing for the arrangement of three iodinated phenyl groups bound thereto.

The invention also relates to the use of such diagnostic compositions as contrast agents in diagnostic imaging and in particular in X-ray imaging and to contrast media containing such compounds.

## Description

### Technical Field of the Invention

The present invention relates to a class of compounds and to diagnostic compositions containing such compounds where the compounds are iodine containing compounds. More specifically the iodine containing compounds are chemical compounds containing a benzene scaffolding moiety allowing for the arrangement of three iodinated phenyl groups bound thereto.

The invention also relates to the use of such diagnostic compositions as contrast agents in diagnostic imaging and in particular in X-ray imaging and to contrast media containing such compounds.

### Description of Related art

All diagnostic imaging is based on the achievement of different signal levels from different structures within the body. Thus in X-ray imaging for example, for a given body structure to be visible in the image, the X-ray attenuation by that structure must differ from that of the surrounding tissues. The difference in signal between the body structure and its surroundings is frequently termed contrast and much effort has been devoted to means of enhancing contrast in diagnostic imaging since the greater the contrast between a body structure and its surroundings the higher the quality of the images and the greater their value to the physician performing the diagnosis. Moreover, the greater the contrast the smaller the body structures that may be visualized in the imaging procedures, i.e. increased contrast can lead to increased spatial resolution.

The diagnostic quality of images is strongly dependent on the inherent noise level in the imaging procedure, and the ratio of the contrast level to the noise level can thus be seen to represent an effective diagnostic quality factor for diagnostic images.

Achieving improvement in such a diagnostic quality factor has long been and still remains an important goal. In techniques such as X-ray, magnetic resonance imaging (MRI) and ultrasound, one approach to improving the diagnostic quality factor has been to introduce contrast enhancing materials formulated as contrast media into the body region being imaged.

Thus in X-ray early examples of contrast agents were insoluble inorganic barium salts which enhanced X-ray attenuation in the body zones into which they distributed. For the last 50 years the field of X-ray contrast agents has been dominated by soluble iodine containing compounds. Commercial available contrast media containing iodinated contrast agents are usually classified as ionic monomers such as diatrizoate (marketed e.g. under the trade name Gastrografen™), ionic dimers such as ioxaglate (marketed e.g. under the trade name Hexabrix™), nonionic monomers such as iohexol (marketed e.g. under the trade name Omnipaque™), iopamidol(marketed e.g. under the trade name Isovue^{™}), iomeprol (marketed e.g. under the trade name Iomeron^{™}) and the non-ionic dimer iodixanol (marketed under the trade name and Visipaque™).

The most widely used commercial non-ionic X-ray contrast agents such as those mentioned above are considered safe. Contrast media containing iodinated contrast agents are used in more that 20 millions of X-ray examinations annually in the USA and the number of adverse reactions is considered acceptable. However, since a contrast enhanced X-ray examination will require up to about 200 ml contrast media administered in a total dose, there is a continuous drive to provide improved contrast media.

The utility of the contrast media is governed largely by its toxicity, by its diagnostic efficacy, by adverse effects it may have on the subject to which the contrast medium is administered, and by the ease of storage and ease of administration. Since such media are conventionally used for diagnostic purposes rather than to achieve direct therapeutic effect, it is generally desirable to provide media having as little as possible effect on the various biological mechanisms of the cells or the body as this will lead to lower toxicity and lower adverse clinical effect. The toxicity and adverse biological effects of a contrast medium are contributed to by the components of the formulation medium, e.g. the solvent or carrier as well as the contrast agent itself and its components such as ions for the ionic contrast agents and also by its metabolites.

The major contributing factors to the toxicity of the contrast medium are identified as the chemotoxicity of the contrast agent, the osmolality of the contrast medium and the ionic composition or lack thereof of the contrast medium.

Desirable characteristics of an iodinated contrast agent are low toxicity of the compound itself (chemotoxicity), low viscosity of the contrast medium wherein the compound is dissolved, low osmolality of the contrast medium and a high iodine content (frequently measured in g iodine per ml of the formulated contrast medium for administration). The iodinated contrast agent must also be completely soluble in the formulation medium, usually an aqueous medium, and remain in solution during storage.

The osmolalities of the commercial products and in particular of the non-ionic compounds is acceptable for most media containing dimers and non-ionic monomers although there is still room for improvement. In coronary angiography for example, injection into the circulatory system of a bolus dose of contrast medium has caused severe side effects. In this procedure contrast medium rather than blood flows through the system for a short period of time, and differences in the chemical and physiochemical nature of the contrast medium and the blood that it replaces can cause undesirable adverse effects such as arrhythmias, QT prolongation and reduction in cardiac contractive force. Such effects are seen in particular with ionic contrast agents where osmotoxic effects are associated with hypertonicity of the injected contrast medium. Contrast media that are isotonic or slightly hypotonic with the body fluids are particularly desired. Low osmolar contrast media have low renal toxicity which is particularly desirable. The osmolality is a function of the number of particles per volume unit of the formulated contrast medium.

To keep the injection volume of the contrast media as low as possible it is highly desirable to formulate contrast media with high concentration of iodine/ml, and still maintain the osmolality of the media at a low level, preferably below or close to isotonicity. The development of non-ionic monomeric contrast agents and in particular non-ionic bis(triiodophenyl) dimers such as iodixanol (EP patent 108638) has provided contrast media with reduced osmotoxicity allowing contrast effective iodine concentration to be achieved with hypotonic solution, and has even allowed correction of ionic imbalance by inclusion of plasma ions while still maintaining the contrast medium Visipaque^{™} at the desired osmolality (WO 90/01194 and WO 91/13636).

However, X-ray contrast media at commercial high iodine concentration have relative high viscosity, ranging from about 15 to about 60 mPas at ambient temperature. Generally, contrast media where the contrast enhancing agent is a dimer has higher viscosity than the corresponding contrast media where the contrast enhancing agent is the monomer corresponding to the dimer. Such high viscosities pose problems to the administrators of the contrast medium, requiring relatively large bore needles or high applied pressure, and are particularly pronounced in pediatric radiography and in radiographic techniques which require rapid bolus administration, e.g. in angiography.

X-ray contrast agents of high molecular weight has been proposed, e.g. polymers with substituted triiodinated phenyl groups grafted on the polymer, see EP 354836, EP 436316 and US 5019370. Further, WO 9501966, EP 782563 and US patent 5817873 read on compounds having e.g. 3 and 4 substituted triiodinated phenyl groups arranged linearly or around a central core. However, none of these proposed compounds are on the market.

Hence there still exists a desire to develop contrast agents that solves one or more of the problems discussed above. Such agents should ideally have improved properties over the soluble iodine containing compounds in one or more of the following properties: renal toxicity, osmolality, viscosity, solubility, injection volumes/iodine concentration and attenuation/radiation dose.

### Summary of the Invention

The present invention provides compounds useful as contrast media having improved properties over the known media with regards to at least one of the following criteria osmolality (and hence the renal toxicity), viscosity, iodine concentration and solubility. The contrast media comprises iodine containing contrast enhancing compounds where the iodine containing compounds are chemical compounds containing a benzene scaffolding moiety, allowing for the arrangement of three iodinated phenyl groups bound thereto. The iodine containing contrast enhancing compounds can be synthesized from commercially available and relatively inexpensive starting materials.

The invention is further described in the attached claims.

### Detailed Description of the Invention

The contrast enhancing compounds are synthetic chemical compounds of formula (I) 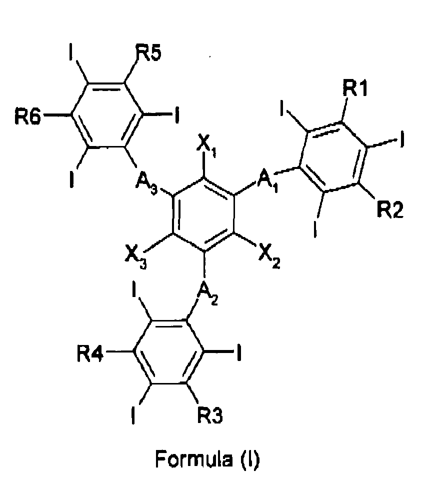 wherein
each of the substituents R¹, R², R³, R⁴, R⁵ and R⁶ (hereinafter collectively denoted R group(s)) may be the same or different and denote a hydrogen atom or a non-ionic hydrophilic moiety, provided that at least one R group is a hydrophilic moiety; and
each of the A¹, A² and A³ denote linker groups (collectively called A groups) that independently of each other denote a single covalent bond or linker groups comprising ester or amide functions or alkylene chains with 1 to 5 carbon atom wherein the alkylene chain can be interrupted by one or more oxygen, nitrogen or sulphur atoms or can contain an ester or amide function; and
each of X¹, X² and X³ (collectively called X groups) independently of each other denote a hydrogen or a iodine atom; and
salts or optical active isomers thereof.

The non-ionic hydrophilic moieties may be any of the non-ionizing groups conventionally used to enhance water solubility. Suitable groups include esters, amides and amine moieties that may be further substituted. Further substituents include straight chain or branched chain C₁₋₁₀ alkyl groups, preferably C₁₋₅ alkyl groups, optionally with one or more CH₂ or CH moieties replaced by oxygen or nitrogen atoms and optionally substituted by one or more groups selected from oxo, hydroxyl, amino or carboxyl derivatives, and oxo substituted sulphur and phosphorus atoms. Particular examples include polyhydroxyalkyl, hydroxyalkoxyalkyl and hydroxypolyalkoxyalkyl and such groups attached to the phenyl group via an amide linkage such as hydroxyalkylaminocarbonyl, N-alkyl-hydroxyalkylaminocarbonyl and bis-hydroxyalkylaminocarbonyl groups.

In a preferred embodiment the hydrophilic moieties R are selected from the groups listed below and preferably containing 1 to 6 hydroxy groups, more preferably 1 to 3 hydroxy groups.

Examples of preferred groups comprise groups of the formulas:
-CONH-CH₂-CH₂-OH
-CONH-CH₂-CHOH-CH₂-OH
-CON(CH₃)CH₂-CHOH-CH₂OH
-CONH-CH-(CH₂ -OH)₂
-CON-(CH₂-CH₂-OH)₂
-CONH₂
-CONHCH₃
-NHCOCH₂OH
-N(COCH₃)H
-N(COCH₃) C₁₋₃ alkyl
-N(COCH₃) - mono, bis or tris-hydroxy C₁₋₄ alkyl
-N(COCH₂OH) - hydrogen, mono, bis or tris-hydroxy C₁₋₄ alkyl
- N(CO-CHOH-CH2OH) - hydrogen, mono, bis or trihydroxylated C₁₋₄ alkyl.
- N(CO-CHOH-CHOH-CH2OH) - hydrogen, mono, bis or trihydroxylated C₁₋₄ alkyl.
-N(COCH₂OH)₂
-CON (CH₂-CHOH-CH₂-OH) (CH₂-CH₂-OH)
-CONH-C (CH₂-OH)₃ and
-CONH-CH (CH₂-OH) (CHOH -CH₂-OH).

More preferably the R groups will be equal or different and denote one or more moieties of the formulas CON(CH₃)CH₂-CHOH-CH₂OH, -CONH-CH₂-CHOH-CH₂-OH, -CONH-CH-(CH₂-OH)₂, -CON-(CH₂-CH₂-OH)₂, -CONH-CH₂-CHOH-CH₂-OH, -NHCOCH₂OH and - N(COCH₂OH) - mono, bis or tris-hydroxy C₁₋₄ alkyl.

Even more preferably, all the R¹, R³ and R⁵ groups are equal and they denote one of these moieties and all the groups R², R⁴ and R⁶ may also be equal but may be different from the R¹, R³ and R⁵ groups. All the R groups may also be equal. Most preferably all R groups are equal and denote one of the preferred moieties and most preferred the moiety -CONH-CH₂-CHOH-CH₂-OH.

The A groups preferably are linker groups with 1 to 5 atoms in the chain, such as an alkylene chain with 1 to 5 carbon atom wherein the alkylene chain can be interrupted by one or more oxygen, nitrogen or sulphur atom and/or can contain an ester or amide function. The A groups can also consist of an ester or amide function as illustrated below. Particularly preferred are groups with 2 or 3 atoms in the linking chain and particularly preferred are ester, amide groups and acetamide groups.

Compounds where one to three of the X groups are hydrogen are also preferred.

Thus examples of preferred structures according to the invention include the compounds of formulas (IIa), (IIb) and (IIc).

In formula (IIa) the X groups in formula (I) denote an hydrogen atom, the A groups are equal and denote a amide bridging moiety and the R groups are the same and denote amide groups of formula -CO-NR'R" wherein at least one of the R' or R" moieties denote an alkyl group, preferably a C₁₋₅ alkyl group and more preferably a C₁₋₃ alkyl group preferably substituted by one or more hydroxy groups; and optionally one of the R' and R" denote a hydrogen atom. Most preferably one of the moieties R' and R" denotes a hydrogen atom and the other denote a 2,3 dihydroxypropyl moiety.

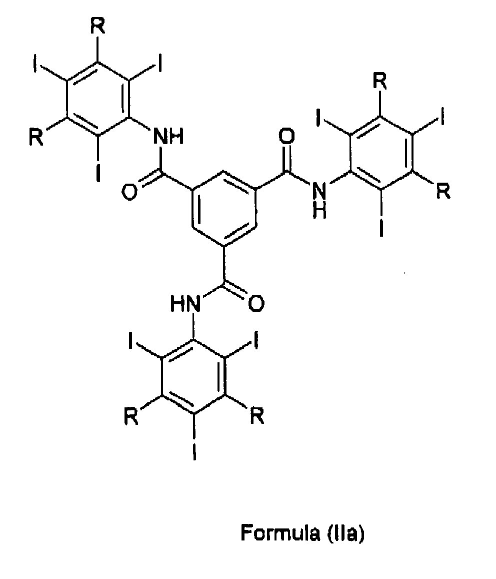

In a further example the preferred structures according to the invention include the compounds of formula (IIb) below. In formula (IIb) the X groups in formula (I) denotes an hydrogen atom, the A groups are the same and denote a acetamide bridging moiety and the R groups are the same and denote amide groups of formula -CO-NR'R" wherein at least one of the R' or R" moieties denote an alkyl group, preferably a C₁₋₅ alkyl group and more preferably a C₁₋₃ alkyl group preferably substituted by one or more hydroxy groups; and optionally one of the R' and R" denote a hydrogen atom. Most preferably one of the moieties R' and R" denotes a hydrogen atom and the other denote a 2,3 dihydroxypropyl moiety.

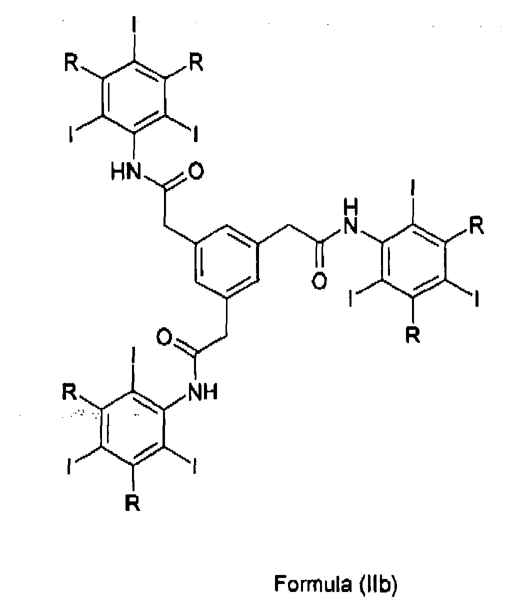

In a still further example the preferred structures according to the invention include the compounds of formula (IIc) below. In formula (IIc) each of the X groups in formula (I) denotes an iodine atom, the A groups are the same and denote a acetamide bridging moiety and the R groups are the same and denote amide groups of formula -CO-NR'R" wherein at least one of the R' or R" moieties denote an alkyl group, preferably a C₁₋₆ alkyl group and more preferably a C₁₋₃alkyl group preferably substituted by one or more hydroxy groups; and optionally one of the R' and R" denote a hydrogen atom. Most preferably one of the moieties R' and R" denotes a hydrogen atom and the other denote a 2,3 dihydroxypropyl moiety.

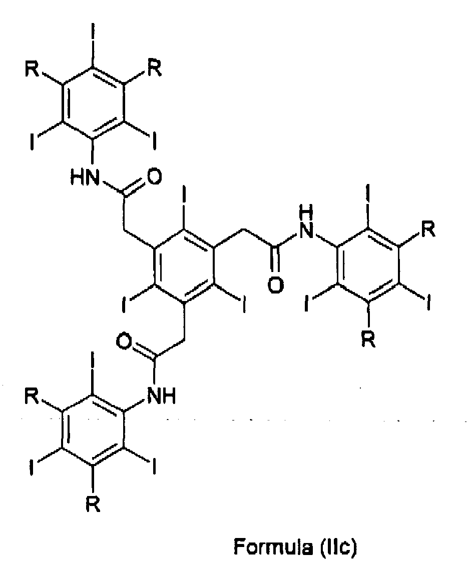

In a particularly preferred example the preferred structures according to the invention include the compounds of formula (III) below. In formula (III) the X groups in formula (I) all denote hydrogen atoms, the A groups are the same and denote a acetamide bridging moiety and the R groups are the same and denote amide groups of formula -CO-NR'R" wherein one of the R' and R" moieties denote hydrogen atoms and the other denotes a 2,3 dihydroxypropyl moiety.

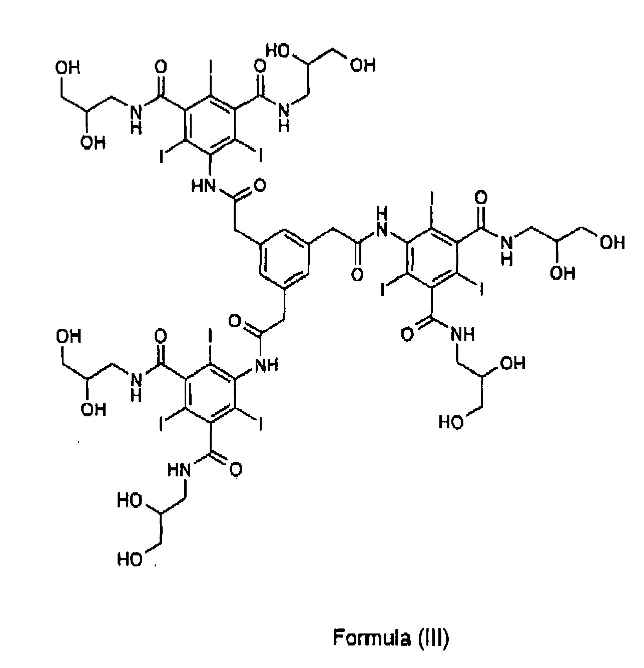

The compounds of formula (I) all have a benzene moiety as the central scaffolding group. The ortho iodine atoms of the iodine substituted phenyl groups will force these phenyl groups out of the ring plane of the central benzene ring, making the molecule adopt a globular form. Globular molecules will usually have enhanced solubility compared with similar molecules with a more planar structure.

At an iodine concentration of 320 mg/ml, which is a common concentration for commercially available iodinated contrast media, the concentration of the compound of formula (I) will be approximately 0.28 M (Molar) when the X groups in formulas denote hydrogen atoms. When the X-groups of formula (I) denote iodine, the concentration will be approximately 0.21 M (Molar).The contrast medium will also be hypoosmolar at this iodine concentration, and this is an advantageous property with regards to the nephrotoxicity of the contrast medium. It is also possible to add electrolytes to the contrast medium to lower the cardiovascular effects as explained in WO 90/01194 and WO 91/13636.

Compounds of formula (I) also comprises optical active isomers. Both enantiomerically pure products as well as mixtures of optical isomers are included.

The compounds of the invention are useful as contrast agents and may be formulated with conventional carriers and excipients to produce diagnostic contrast compositions.

Thus viewed from a further aspect the invention provides a diagnostic agent comprising a compound of formula (I) and a diagnostic composition comprising a compound of formula (I) as described above together with at least one physiologically tolerable carrier or excipient, e.g. in aqueous solution for injection optionally together with added plasma ions or dissolved oxygen. The diagnostic agent and composition preferably are X-ray diagnostic agents and compositions.

In a still further aspect the invention comprises the use of a diagnostic agent and a diagnostic composition containing a compound of formula (I) as defined above in contrast enhanced examination such as X-ray contrast examinations, and also the use of a compound of formula (I) for the manufacture of a diagnostic composition for use as a contrast agent, e.g. a X-ray contrast agent.

The compounds of formula (I) are also useful in a method of diagnosis of a human or non-human animal preadministered with a compound of formula (I) which comprising examining the body with a diagnostic device and compiling data from the examination, and also in a method of diagnosis which comprises administration of compounds of formula (I) to the human and non-human animal body and conducting the examination as mentioned.

Within the scope of the invention is also a method of imaging, specifically X-ray imaging which comprises administration of compounds of formula (I) to the human or non-human animal body, examining the body with a diagnostic device and compiling data from the examination and optionally analysing the data.

The contrast agent composition of the invention may be in a ready to use concentration or may be in a concentrate form for dilution prior to administration. Generally compositions in a ready to use form will have iodine concentrations of at least 100 mg l/ml, preferably at least 150 mg l/ml, with concentrations of at least 300 mg l/ml, e.g. 320 mg l/ml being preferred. The higher the iodine concentration, the higher is the diagnostic value in the form of X-ray attenuation of the contrast media. However, the higher the iodine concentration the higher is the viscosity and the osmolality of the composition. Normally the maximum iodine concentration for a given contrast media will be determined by the solubility of the contrast enhancing agent, e.g. the iodinated compound, and the tolerable limits for viscosity and osmolality.

For contrast media which are administered by injection or infusion, the desired upper limit for the solution's viscosity at ambient temperature (20°C) is about 30mPas, however viscosities of up to 50 to 60 mPas and even more than 60 mPas can be tolerated. For contrast media given by bolus injection, e.g. in angiographic procedures, osmotoxic effects must be considered and preferably the osmolality should be below 1 Osm/kg H₂O, preferably below 850 mOsm/kg H₂O and more preferably about 300 mOsm/kg H₂O.

With the compounds of the invention such viscosity, osmolality and iodine concentrations targets can be met. Indeed, effective iodine concentrations can be reached with hypotonic solutions. It may thus be desirable to make up the solution's tonicity by the addition of plasma cations so as to reduce the toxicity contribution that derives from the imbalance effects following bolus injection. Such cations will desirably be included in the ranges suggested in WO 90/01194 and WO 91/13636.

In particular, addition of sodium and calcium ions to provide a contrast medium isotonic with blood for all iodine concentrations is desirable and obtainable. The plasma cations may be provided in the form of salts with physiologically tolerable counterions, e.g. chloride, sulphate, phosphate, hydrogen carbonate etc., with plasma anions preferably being used.

The compounds of the general formula (I) can be synthesized by several synthetic pathways known or obvious to the skilled artisan. In one embodiment, a substituted benzene moiety comprising a 1, 3, 5 substituted benzene moiety optionally substituted with iodine atoms in the 2, 4 and/or 6 positions is provided. The 1, 3 or 5 positions contain reactive moieties, e.g. acidic functions such as an acid chloride group, or amine groups. The compounds can be processed from commercially available materials such as from trimesic acid or from 1,3,5-triaminobenzene.

The triiodinated phenyl groups each substituted by two R groups likewise contains one reactive moiety at the phenyl group. The reactive moieties will then by reaction form linker groups A and three iodinated phenyl groups are linked to the central benzene scaffolding group by this addition reaction. When the R groups contain reactive groups, e.g. hydroxyl groups, such groups should be protected e.g. by acylation, preferably by acetylation. Tri-iodinated phenyl groups are commercially available or can be produced following procedures described or referred to e.g. in WO95/35122 and WO98/52911. The preferred tri-iodinated compound 5-amino-2,4,6-triiodo-N,N'-bis(2,3-dihydroxypropyl)-isophtalamide is also commercially available e.g. from Fuji Chemical Industries, Ltd.

The general procedure can be illustrated by the scheme below, where a compound of formula (IIa) is produced: 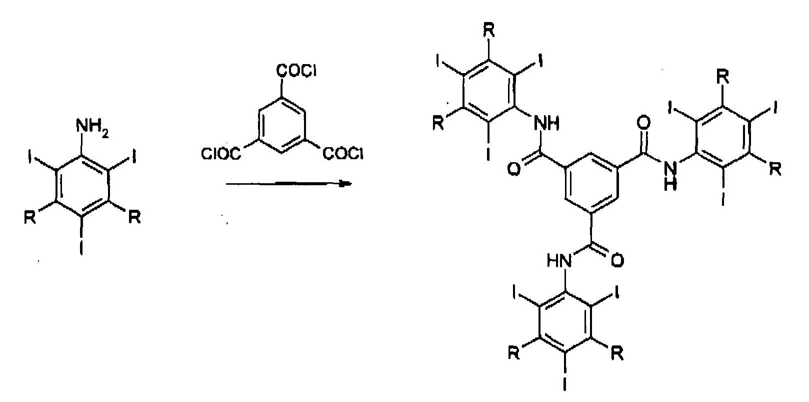 wherein the R groups have the meaning denoted above.

### Example:

### 1,3,5-N,N',N''-Tris-[3,5-N,N'-bis-(2,3-dihydroxypropyl)-carbamido-2,4,6-triiodophenyl]-triacetemidobenzene.

### a: 1,3,5-Benzenetriacetyl chloride.

To 1,3,5-benzenetriacetic acid (2.5 g, 9.9 mmol, from Fluka) was added a drop of N,N-dimethylformamide followed by slow addition of thionyl chloride (5.41 ml, 74 mmol) at ambient temperature and with good stirring. The mixture was heated to 75°C and held there for 2 h (gas evolution occurred when heating above 50°C). The mixture was cooled and evaporated to dryness. The residue was coevaporated twice with 1,1,1-trichloroethane (2x 15 ml). A red oil was left. Yield 3.0 g (= 98%).
¹H NMR (CDCl₃): 7.17 (s, 3H), 4.18 (s, 6H).
The product was used without further purification.

### b: 5-Amino-1,3-N.N'-bis-(2,3-diacetoxypropyl)-2,4,6-triiodoisophtalamide.

5-Amino-1,3-N,N'-bis-(2,3-dihydroxypropyl)-2,4,6-triiodoisophtalamide (40.0 g, 56.7 mmol) was suspended in pyridine (80 ml). With effective stirring and cooling in an ice/water bath, acetic anhydride (70 ml) was added dropwise. After addition the mixture was stirred at ambient temperature over night. The clear solution was evaporated to an oil, which was dissolved in ethyl acetate (450 ml). This solution was washed with diluted hydrochloric acid (0.05 M, 100 ml), water (200 ml), diluted solution of sodium hydrogen carbonate (5 %, 2 × 100 ml) and at last a saturated solution of sodium chloride (70 ml). The organic phase was dried (NaSO₄) and the solvent evaporated to a white crystalline substance. Yield 49.5 g (= quantitative).
¹H NMR (CDCl3): 6.71 & 6.38 (two t:s, 2H), 5.24 (br. s, 2H), 5.09 (s, 2H), 4.37 - 4.48 & 4.22 - 4.35 (m:s, 4H), 3.71 - 3.85 & 3.50 - 3.65 (m:s, 4H), 2.06 (s, 12H).

### c: 1,3,5-N,N',N''-Tris-[3,5-N,N'-bis-(2,3-diacetoxypropyl)-carbamido-2,4,6-triiodophenyl]-triacetamidobenzene.

5-Amino-1,3-N,N'-bis-(2,3-diacetoxypropyl)-2,4,6-triiadoisophtalamide (4.9 g, 41 mmol) was dissolved in dry N,N-dimethylacetamide (2.5 ml) at ambient temperature. With efficient stirring 1,3,5-benzenetriacetyl chloride (0.25 g, 1 mmol) dissolved in methylene chloride (0.5 ml) was added drop-wise. The mixture was stirred at ambient temperature and under inert atmosphere for 3 days. The mixture was then evaporated in vacuo to an oil. This oil was trituated three times with an acetonitrile/water mixture (50/50, 3 × 10 ml). A white to tan coloured residue was left, which was pure according to HPLC. Yield 0.92 g ( = 33%).
MS (ES⁺, m/e): 2817 ([M]⁺, 100%)
¹H NMR (DMSO-d₆): 10.18 & 10.16 (two s:s, 3H), 8.40 - 8.95 (m, 6H), 7.33 (s, 3H), 5.07 (br. s, 6H), 4.12 - 4.36 (two m:s, 12H), 3.34 - 3.52 (m:s, 12H), 2.06 (s, 36H).

### d: 1,3,5-N,N',N"-Tris-[3,5-N,N'-bis-(2,3-dihydroxypropyl)-carbamido-2,4,6-triiodophenyl]- triacetamidobenzene.

1,3,5-N,N',N"-Tris-[3,5-N,N"-bis-(2,3-diacetoxypropyl)-carbamido-2,4,6-triiodophenyl]triaoetamidobenzene (2.0 g, 7.1 mmol) was suspended in tetrahydrofuran (250 ml) in a flask. The suspension was saturated with gaseous methylamine and the flask was closed and the contents stirred at ambient temperature for two days. The mixture was then evaporated to dryness and the residue trituated with chloroform (2× 20 ml) and the with a mixture of acetonitrile / water (5/1, 2 × 10 ml). A white powder was left. Yield 1.59 g (= 97%).
MS (ES⁺, m/e): 2313 ([M]⁺, 14%), 2295 ([M-H₂O]⁺, 100%).
¹H NMR (DMSO-d₆): 10.17 (br. s, 3H), 8.40 - 8.64 (m, 6H), 7.33 (s, 3H), 4.67 -4.78 (br. s:s, 6H), 4.55 (br. s, 6H), 4.02 & 3.53 & 3.41 (unres. m:s, 18H), 3.25 &
3.18 ( s + m, 18H).
¹³C NMR (DMSO-d₆): 169.7, 168.5, 150.1, 135.1, 129.5, 118.1, 99.4, 86.3, 85.2, 70.0, 64.0, 60.3, 42.7.

## Claims

1. Compounds of formula (I) 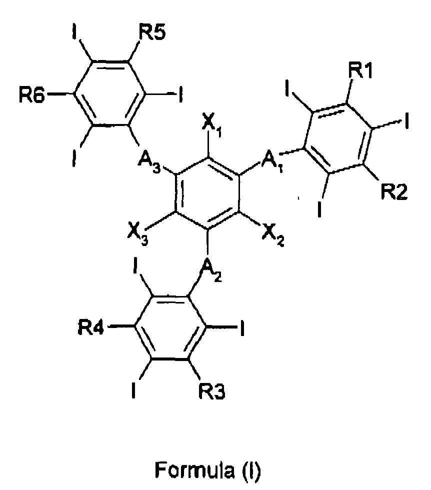 wherein
each of the substituents R¹, R², R³, R⁴, R⁵ and R⁶ are the same or different and denote a hydrogen atom or a non-ionic hydrophilic moiety, provided that at least one of the groups is a hydrophilic moiety;
each of A¹, A² and A³ independently of each other denote a single covalent bond or denote linker groups comprising an ester or amide function or an alkylene chain with 1 to 5 carbon atom which can be interrupted by one or more oxygen, nitrogen or sulphur atoms or can contain an ester or amide function:
each of X¹, X² and X³ independently of each other denote a hydrogen atom or a iodine atom; and
salts or optical active isomers thereof.

2. Compounds as claimed in claim 1 wherein each of R¹, R², R³, R⁴, R⁵ and R⁶ denote esters, amides and amine moieties, optionally further substituted by a straight chain or branched chain C₁₋₁₀ alkyl groups, preferably C₁₋₅ alkyl groups, optionally with one or more CH₂ or CH moieties replaced by oxygen or nitrogen atoms and optionally substituted by one or more groups selected from oxo, hydroxyl, amino or carboxyl derivative, and oxo substituted sulphur and phosphorus atoms.

3. Compounds as claimed in claims 1 to 2 wherein each of R¹, R², R³, R⁴, R⁵ and R⁶ are hydrophilic moieties containing 1 to 6 hydroxy groups, preferably 1 to 3 hydroxy groups.

4. Compounds as claimed in any of the preceding claims wherein each of R¹, R², R³, R⁴, R⁵ and R⁶ may be the same or different and are polyhydroxyalkyl, hydroxyalkoxyalkyl and hydroxypolyalkoxyalky groups attached to the phenyl group via an amide linkage such as hydroxyalkylaminocarbonyl, N-alkyl-hydroxyalkylaminocarbonyl and bis-hydroxyalkylaminocarbonyl groups.

5. Compounds as claimed in claims 1 to 3 wherein each of R¹, R², R³, R⁴, R⁵ and R⁶ are the same or different and are selected from groups of the formulas
- CONH-CH₂-CH₂-OH
- CONH-CH₂-CHOH-CH₂-OH
- CON(CH₃)CH₂-CHOH-CH₂OH
- CONH-CH-(CH₂-OH)₂
- CON-(CH₂-CH₂-OH)₂
- CONH₂
- CONHCH₃
- NHCOCH₂OH
- N(COCH₃)H
- N(COCH₃) C₁₋₃ alkyl
- N(COCH₃) - mono, bis or tris-hydroxy C₁₋₄ alkyl
- N(COCH₂OH) - hydrogen, mono, bis or tris-hydroxy C₁₋₄ alkyl
- N(CO-CHOH-CH2OH) - hydrogen, mono, bis or trihydroxylated C₁₋₄ alkyl.
- N(CO-CHOH-CHOH-CH2OH) - hydrogen, mono, bis or trihydroxylated C₁₋₄ alkyl.
- N(COCH₂OH)₂
- CON (CH₂CHOH-CH₂-OH) (CH₂-CH₂-OH)
- CONH-C (CH₂-OH)₃ and
- CONH-CH (CH₂-OH) (CHOH -CH₂-OH).

6. Compounds as claimed in claim 5 wherein each of R¹, R², R³, R⁴, R⁵ and R⁶ may be equal or different and are selected from one or more moieties of the formulas CON(CH₃)CH₂-CHOH-CH₂OH, -CONH-CH₂-CHOH-CH₂-OH, - CONH-CH-(CH₂-OH)₂, -CON-(CH₂-CH₂-OH)₂, -CONH-CH₂-CHOH-CH₂-OH, - NHCOCH₂OH and - N(COCH₂OH) - mono, bis or tris-hydroxy C₁₋₄ alkyl.

7. Compound as claimed in claim 6 wherein each of R¹, R², R³, R⁴, R⁵ and R⁶ are equal and are -CONH-CH₂-CHOH-CH₂-OH.

8. Compound as claimed in any of the preceding claims wherein each of A¹, A² and A³ denote linker groups with 1 to 5 atoms in the chain.

9. Compound as claimed in claim 8 wherein each of linker groups denote an alkylene chain with 1 to 5 carbon atoms optionally interrupted by one or more oxygen, nitrogen or sulphur atom and/or containing an ester or amide function.

10. Compound as claimed in claim 8 wherein each of the linker groups consists of an ester or amide or acetamide group.

11. Compound as claimed in any of the preceding claims wherein X¹, X² and X³ denote a hydrogen atom.

12. Compounds as claimed in any of the preceding claims of the formulas (IIa), (IIb) and (IIc) 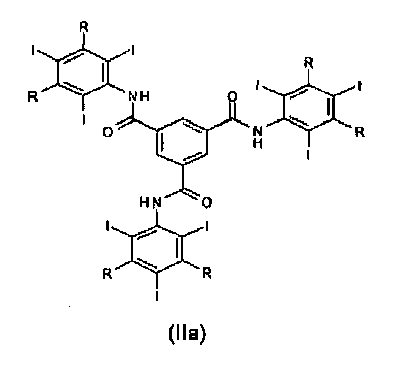 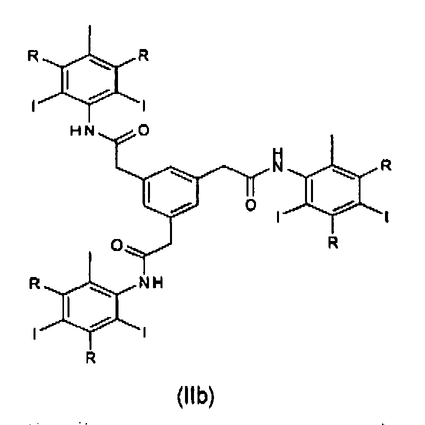 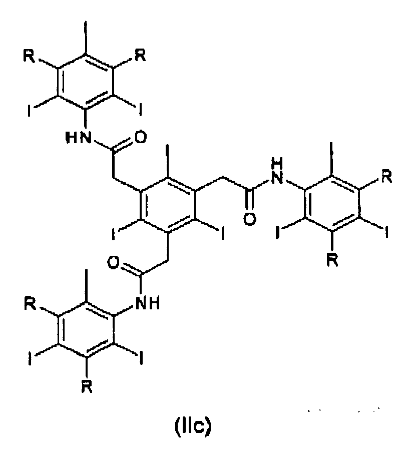 wherein in formulas (IIa), (IIb) and (IIc) the R groups are the same and denote amide groups of formula -CO-NR'R" wherein at least one of the R' or R" moieties denote an alkyl group, preferably a C₁₋₅ alkyl group and more preferably a C₁₋₃ alkyl group preferably substituted by one or more hydroxy groups; and optionally one of the R' and R" denote a hydrogen atom.

13. Compound as claimed in any of the preceding claims being 1,3,5-N.N',N''-Tris-[3,5-N,N'-bis-(2,3-dihydroxypropyl)-carbamido-2,4,6-trilodophenyl]-triacetamidobenzene.

14. A diagnostic agent comprising a compound of formula (I) as defined in any of the preceding claims

15. A diagnostic composition comprising a compound of formula (I) as defined in claims 1 to 13 together with a pharmaceutically acceptable carrier or excipient and optionally together with added plasma ions and/or dissolved oxygen.

16. An X-ray diagnostic composition comprising a compound of formula (I) as defined in claims 1 to 13 together with a pharmaceutically acceptable carrier or excipient.

17. Use of a diagnostic agent and a diagnostic composition containing a compound of formula (I) as defined in claims 1 to 13 in contrast enhanced examinations.

18. Use of a compound of formula (I) as defined in claims 1 to 13 for the manufacture of a diagnostic composition for use as a contrast agent, in particular as a X-ray contrast agent.

19. A method of diagnosis of a human or non-human animal preadministered with a compound of formula (I) as defined in claims 1 to 13, comprising examining the body with a diagnostic device and compiling data from the examination.

20. A method of imaging, specifically X-ray imaging, comprising administration of compounds of formula (I) as defined in claims 1 to 13 to the human or non-human animal body, examining the body with a diagnostic device and compiling data from the examination and optionally analysing the data.
